# EUROPÄISCHE PATENTSCHRIFT

(11) **EP 3 352 901 B1**
(45) Veröffentlichungstag und Bekanntmachung des Hinweises auf die Patenterteilung: **07.08.2019**
(21) Anmeldenummer: 16770888.2
(22) Anmeldetag: 16.09.2016
(51) Int. Cl.: B01J 31/12, C07D 307/77

(54) **BISMUTVERBINDUNGEN ENTHALTEND PERFLUORALKYLGRUPPEN ALS LEWIS-SÄURE KATALYSATOREN**
PERFLUOROALKYL GROUP-CONTAINING BISMUTH COMPOUNDS AS LEWIS ACID CATALYSTS
COMPOSÉS BISMUTH CONTENANT DES GROUPES PERFLUOROALKYLE EN TANT QUE CATALYSEURS SOUS FORME D'ACIDES DE LEWIS

(30) Priorität: 23.09.2015 DE 102015012193
(43) Veröffentlichungstag der Anmeldung: 01.08.2018
(73) Patentinhaber: Merck Patent GmbH, 64293 Darmstadt (DE)
(72) Erfinder: HÖGE, Berthold Theo, 33604 Bielefeld (DE); SOLYNTJES, Sven Joerg-Ruediger August, 33613 Bielefeld (DE); BADER, Anne Julia, 33615 Bielefeld (DE); IGNATIEV, Nikolai, 47058 Duisburg (DE)
(86) Internationale Anmeldenummer: PCT/EP2016/001557
(87) Internationale Veröffentlichungsnummer: WO 2017/050425

(56) Entgegenhaltungen:
- EP-A1- 1 344 772
- THIERRY OLLEVIER: "New trends in bismuth-catalyzed synthetic transformations", ORGANIC & BIOMOLECULAR CHEMISTRY, Bd. 11, Nr. 17, 1. Januar 2013 (2013-01-01), Seite 2740, XP055327916, GB ISSN: 1477-0520, DOI: 10.1039/c3ob26537d in der Anmeldung erwähnt
- DIETER NAUMANN ET AL: "The preparations and properties of tris(perfluoroorgano)bismuth compounds Bi(Rf)3 (Rf = CF3, C2F5, n-C3F7, n-C4F9, n-C6F13, n-C8 F17, C6F5)", JOURNAL OF ORGANOMETALLIC CHEMISTRY., Bd. 334, Nr. 3, 1. November 1987 (1987-11-01), Seiten 323-328, XP055327901, CH ISSN: 0022-328X, DOI: 10.1016/0022-328X(87)80094-3 in der Anmeldung erwähnt
- KIRIJ N V ET AL: "Synthesis and characterization of aryl(trifluoromethyl)bismuth compounds (R-C6H4)3-nBi(CF3)n [R=H, 4-CH3, 4-CF3, 3-F, 4-F; n=1 and 2] and the reactions of (C6H5)3-nnBi(CF3)n [n=0-3] with benzoylpyridinium chloride", JOURNAL OF FLUORINE CHEMISTRY, ELSEVIER, NL, Bd. 69, Nr. 3, 1. Dezember 1994 (1994-12-01), Seiten 219-223, XP026635699, ISSN: 0022-1139 [gefunden am 1994-12-01] in der Anmeldung erwähnt
- DIETER NAUMANN ET AL: "An alternative route for the synthesis of tris(trifluoromethyl)bismuth, Bi(CF3)3", JOURNAL OF FLUORINE CHEMISTRY, Bd. 66, Nr. 1, 1. Januar 1994 (1994-01-01) , Seiten 79-80, XP055327889, NL ISSN: 0022-1139, DOI: 10.1016/0022-1139(93)02899-P in der Anmeldung erwähnt
- GOWRAVARAM SABITHA ET AL: "BiCl 3 -Catalyzed Diastereoselective Intramolecular [4+2] Cycloaddition Reactions Leading to Pyrazole Annulated New Sulfur Heterocycles", SYNTHETIC COMMUNICATIONS, Bd. 33, Nr. 17, 1. September 2003 (2003-09-01), Seiten 3063-3070, XP055328268, PHILADELPHIA, PA; US ISSN: 0039-7911, DOI: 10.1081/SCC-120022482

## Beschreibung

Die Erfindung betrifft Bismutverbindungen enthaltend Perfluoralkylgruppen als Lewis-Säure-Katalysatoren, spezielle Verbindungen sowie deren Verfahren zur Herstellung.

Die Katalyse mit Lewis-Säuren ist eine weit verbreitete Methodik in der Organischen Synthese und für die industrielle Herstellung verschiedener Substanzen von herausragender Bedeutung. Zu den zahlreichen wichtigen industriellen Prozessen, die durch Lewis-Säuren katalysiert werden, gehören zum Beispiel Friedel-Crafts-Alkylierungen und -Acylierungen von Aromaten, Gattermann-Koch-Reaktionen, Beckmann- und Fries-Umlagerungen, Mukaiyama-Aldol-Kondensationen [Acid Catalysis in Modern Organic Synthesis, H. Yamamoto and K. Ishihara (Eds.), WILEY-VCH, Weinheim, 2008].

G. N. Lewis definiert eine Säure als Substanz, die als Elektronenpaarakzeptor agieren kann. Dieser Definition entsprechend sind Lewis-Säuren Elektronenmangelmoleküle bzw. -spezies. Die üblicherweise verwendeten Lewis-sauren Katalysatoren wie AlCl₃, TiCl₄, ZnCl₂ und BF₃-Diethyletherat sind feuchtigkeitsempfindlich und können in der Regel nach Beendigung der Reaktion nicht zurückgewonnen werden.

Weitere bekannte Lewis-Säuren sind Bismutsalze wie BiCl₃, BiBr₃ und Bi(OSO₂CF₃)₃, wie in T. Ollevier, org. Biomol. Chem., 2013, 11, 2740-2755 beschrieben. Bei Verwendung von BiCl₃ als Katalysator ist jedoch eine hohe Beladung notwendig, in der Regel 10 mol%, und die Freisetzung von HCl kann zu Korrosion der Reaktionsappartur führen, falls beispielsweise Stahlgefäße verwendet werden. Der am meisten bekannte Bismut-Katalysator ist Bismuttriflat. Der Nachteil dieser Verbindung ist die Hydrolyseempfindlichkeit bei Anwesenheit von Wasser und dessen Reaktivität mit Alkoholen und Aminen.
F.H.A. Kwie et al, Synthetic Communications, 40, 2010, 1082-1087 oder T.C. Wabnitz et al, Chem. Eur. J. 10, 2004, 484-493 beschreiben, dass eventuell die aus dem Bismuttriflat freigesetzte Trifluormethansulfonsäure (CF₃SO₃H) für die Katalyse verantwortlich sein könnte.

In S. Kobayashi et al, Chem. Eur. J., 12, 2006, 5954-5960 wird vorgeschlagen, dass Bi(III)-Salze durch Verwendung von organischen Liganden, wie beispielsweise 2,2'-Bipyridinderivaten stabilisiert werden könnte.

R. Qiu et al, Adv. Synth. Catal, 352, 2010, 153 berichten über die Verwendung von Bismutperfluoroctansulfonaten als Katalysatoren.

Es besteht deshalb auch weiterhin ein Bedarf nach alternativen Lewis-Säure-Katalysatoren, damit die Lewis-Säure-katalysierten Reaktionen optimal durchgeführt werden können.

Die Aufgabe der vorliegenden Erfindung ist daher alternative Lewis-Säure-Katalysatoren zu entwickeln, welche eine Reaktionsführung der zu katalysierenden Reaktionen in guter Ausbeute ermöglichen.

Überraschenderweise wurde gefunden, dass spezielle Bismutverbindungen, die Perfluoralkylgruppen enthalten, katalytisch aktiv sind und zwar katalytisch aktiver als BiCl₃.

Aus W. Tyrra et al, Can. J. Chem., 1989, 67, 1949-1951 bzw. D. Naumann et al, J. Organomet. Chem., 1987, 334, 323-328 sind die Bismutverbindungen Bi(CF₃)₃, Bi(CF₃)₂Cl, Bi(CF₃)Cl₂, Bi(CF₃)₂F, Bi(C₂F₅)₃, Bi(*n*-C3F7)3, Bi(*n*-C₄F₉)₃, Bi(*n*-C₆F₁₃)₃ und Bi(*n*-C₈F₁₇)₃ bekannt. Eine Anwendung dieser Verbindungen wurde nicht beschrieben.

Aus S. Pasenok et al, J. Organomet. Chem., 1991, 417, C47-C49 sind die Verbindungen Diphenyl(trifluormethyl)bismut und Phenylbis(trifluormethyl)bismut bekannt. Die Verbindungen werden durch Reaktion von Cd(CF₃)₂·2 CH₃CN mit Phenylbismuthalogeniden hergestellt. Die Verbindungen werden als luftempfindlich beschrieben.

In N.V. Kirij et al, J. Fluorine Chem., 1994, 69, 219-223 werden ebenfalls Aryl(trifluormethyl)bismut-Verbindungen beschrieben, in denen die Arylgruppe variiert wurde, sowie deren Reaktion mit Benzoylpyridiniumchlorid.

Der erste Gegenstand der Erfindung ist daher die Verwendung von mindestens einer Verbindung der Formel (I)

(CₘF₂ₘ₊₁)ₙBiX₃₋ₙ (I),

wobei
m jeweils unabhängig voneinander 2, 3 oder 4 bedeutet;
n 1, 2 oder 3 bedeutet und
X F, Cl, Br oder OSO₂CF₃ bedeutet
als Lewis-Säure-Katalysator.

In einer bevorzugten Ausführungsform der Erfindung ist die Perfluoralkylgruppe (CₘF₂ₘ₊₁) bevorzugt bei jedem Auftreten gleich.

Der Gegenstand der Erfindung ist daher weiterhin die Verwendung von mindestens einer Verbindung der Formel (I), wie zuvor beschrieben, wobei die Perfluoralkylgruppe (CₘF₂ₘ₊₁) bei jedem Auftreten gleich ist.

Die Perfluoralkylgruppe (CₘF₂ₘ₊₁), wobei m 2, 3 oder 4 bedeutet, ist vorzugsweise Pentafluorethyl, *n*-Heptafluorpropyl, *iso-*Heptafluorpropyl, *n-*Nonafluorbutyl, sec-Nonafluorbutyl oder *tert*-Nonafluorbutyl. Die Perfluoralkylgruppe (CₘF₂ₘ₊₁) steht besonders bevorzugt für Pentafluorethyl oder *n*-Nonafluorbutyl.

Der Gegenstand der Erfindung ist daher weiterhin die Verwendung von mindestens einer Verbindung der Formel (I), wie zuvor und bevorzugt beschrieben, wobei die Perfluoralkylgruppe (CₘF₂ₘ₊₁) Pentafluorethyl oder *n*-Nonafluorbutyl bedeutet.

In einer weiteren bevorzugten Ausführungsform der Erfindung werden Verbindungen der Formel (I) verwendet, in denen n 1 oder 2 ist. Bevorzugte Verbindungen der Formel (I) in der Verwendung als Lewis-Säure-Katalysatoren sind Verbindungen, in denen X Cl bedeutet.

Der Gegenstand der Erfindung ist daher weiterhin die Verwendung von mindestens einer Verbindung der Formel (I), wie zuvor und bevorzugt beschrieben, wobei X Cl bedeutet.

Ein weiterer Gegenstand der Erfindung sind auch die Verbindungen der Formel (I)

(CₘF₂ₘ₊₁)ₙBiX₃₋ₙ (I),

wobei
m jeweils unabhängig voneinander 2, 3 oder 4 bedeutet;
n 1 oder 2 bedeutet und
X F, Cl, Br oder OSO₂CF₃ bedeutet.
Bevorzugte Verbindungen der Formel (I), in denen n 1 oder 2 bedeutet, sind Verbindungen, in denen X Cl oder F bedeutet.

Besonders bevorzugte Verbindungen der Formel (I), in denen n 1 oder 2 bedeutet, sind Verbindungen, in denen X Cl bedeutet.

Ein weiterer Gegenstand der Erfindung sind daher die Verbindungen der Formel (I), wie zuvor beschrieben, dadurch gekennzeichnet, dass X Cl bedeutet.

Besonders bevorzugte Verbindungen sind Verbindungen der Formel (I), in denen X Cl bedeutet und m 2 oder 4 bedeutet. Ganz besonders bevorzugte Verbindungen sind Verbindungen der Formel (I), in denen X Cl bedeutet und m 2 bedeutet

Ganz besonders bevorzugte Lewis-Säure-Katalysatoren sind die Verbindungen Bi(C₂F₅)₃, Bi(C₂F₃)Cl₂ und Bi(C₂F₅)₂Cl.

Die Verbindungen der Formel (I), in denen n 3 bedeutet, können basierend auf bekannten Synthesemethoden hergestellt werden, beispielsweise unter Verwendung von Cadmium-Komplexen, wie in D. Naumann et al, J. Organomet. Chem., 1987, 334, 323-328 beschrieben, oder Zink-Komplexen, wie in D. Naumann et al, J. Fluorine Chem. 1994, 66 (1), 79-80 beschrieben.

Cadmium-Komplexe sind jedoch wirtschaftlich gesehen unvorteilhafte Startmaterialien.

Ein weiterer Gegenstand der Erfindung ist daher ein Verfahren zur Herstellung von Verbindungen der Formel (I), wie zuvor beschrieben, wobei n 3 bedeutet, dadurch gekennzeichnet, dass eine Verbindung der Formel (II)

(CₘF₂ₘ₊₁)ₙLi (II),

wobei
m 2, 3 oder 4 bedeutet, mit Bismuttrichlorid, Bismuttribromid oder Bismuttristriflat, vorzugsweise mit Bismuttrichlorid, umgesetzt wird, wobei die Bedingungen der Umsetzungen derart gewählt werden, dass sowohl der Wassergehalt als auch der Sauerstoffgehalt maximal 100 ppm betragen.

Die Umsetzung, wie zuvor beschrieben oder nachfolgend beschrieben, findet in einer Inertgasatmosphäre statt, deren Sauerstoffgehalt maximal 100 ppm ist. Es ist besonders bevorzugt, wenn der Sauerstoffgehalt kleiner 100 ppm ist, ganz besonders bevorzugt maximal 50 ppm ist.
Der Wassergehalt der Reagenzien sowie der Inertgasatmosphäre beträgt maximal 100 ppm. Es ist besonders bevorzugt, wenn der Wassergehalt der Reagenzien sowie der Atmosphäre kleiner 100 ppm ist, ganz besonders bevorzugt 5 bis 50 ppm ist.
Die Bedingungen hinsichtlich des Wassergehalts und des Sauerstoffgehalts gelten nicht für die Aufarbeitung nach erfolgreicher Umsetzung von Verbindungen der Formel (II) bzw. der Verbindungen der Formel (lila) und (IIIb), wie nachfolgend beschrieben.

Alternativ zu Verbindungen der Formel (II) können Grignardreagenzien verwendet werden, die beispielsweise der Formel (IIA) entsprechen,

(CₘF₂ₘ₊₁)MgY (IIA),

wobei
m 2, 3 oder 4 bedeutet und Y I oder Br bedeutet. Die für die Umsetzung mit Verbindungen der Formel (II) genannten Reaktionsbedingungen, dass sowohl der Wassergehalt als auch der Sauerstoffgehalt maximal 100 ppm betragen soll, gelten entsprechend auch für die Umsetzung der Verbindungen der Formel (IIA).

Die Verbindungen der Formel (IIA) können basierend auf bekannten Synthesemethoden hergestellt werden.

Bismuttrichlorid, Bismuttribromid und Bismuttristriflat sind kommerziell erhältlich.

Die Verbindungen der Formel (II) können basierend auf bekannten Synthesemethoden hergestellt werden.
Eine Variante der Synthese der Verbindungen der Formel (II) ist die Umsetzung des entsprechenden Monohydrido-Perfluoralkans (HCₘF₂ₘ₊₁) mit einer 2M Lösung von n-Butyllithium in Pentan in trockenem Diethylether, wie im Beispielteil beschrieben.
Alternativ kann eine Verbindung der Formel (II) aus Perfluoralkyliodiden hergestellt werden, wie in N. Yu. Adonin et al, Z. Anorg. Allg. Chem., 2007, 633, 647-652 beschrieben.
Für die weitere Reaktion der Verbindung der Formel (II), wie zuvor beschrieben, mit Bismuttrichlorid, Bismuttribromid oder Bismuttristriflat, wird bevorzugt ohne weitere Aufreinigung umgesetzt.

Alternativ können die Verbindungen der Formel (II) durch Umsetzung eines Tris(perfluoralkyl)phosphins (P(CmF₂ₘ₊₁)₃) mit einer 1.6M Lösung von *n-*Butyllithium in Hexan hergestellt werden, wie im Beispielteil beschrieben. Für die weitere Reaktion der Verbindung der Formel (II), wie zuvor beschrieben, mit Bismuttrichlorid, Bismuttribromid oder Bismuttriflat, wird bevorzugt ohne weitere Aufreinigung umgesetzt.

Die Umsetzung mit Bismuttrichlorid erfolgt direkt nach der *in situ* Herstellung der Verbindung der Formel (II), wie zuvor beschrieben, in dem vorhandenen Lösemittel oder Lösemittelgemisch.
Bevorzugt werden die Startmaterialien bei tiefer Temperatur, beispielsweise bei Temperaturen zwischen -100°C und -65°C gemischt. Dann wird das Gemisch bevorzugt auf eine Temperatur zwischen -10°C und 5°C erwärmt, vorzugsweise auf 0°C. Die Reaktionsmischung wird dann unter Inertgasatmosphäre filtriert.

Es schließt sich bevorzugt eine geeignete Aufreinigungsmethode an. Beispielsweise Waschen des Rückstands mit einem geeigneten Lösemittel und anschließender Sublimation.

Ein weiterer Gegenstand der Erfindung ist ein Verfahren zur Herstellung von Verbindungen der Formel (I), wobei n 1 oder 2 bedeutet, dadurch gekennzeichnet, dass eine Verbindung der Formel (II)

(CₘF₂ₘ₊₁)ₙLi (II),

wobei
m jeweils unabhängig voneinander 2, 3 oder 4 bedeutet, in einer ersten Umsetzung mit Dichlorarylbismutan oder Chlor(diaryl)bismutan umgesetzt wird, wobei Aryl im ensprechenden Bismutan eine Arylgruppe mit 6 bis 10 C-Atomen bedeutet, die substituiert oder unsubstituiert sein kann,
und nachfolgend die Zwischenprodukte der Formel (IIIa) oder (IIIb)

ArBi(CₘF₂ₘ₊₁)₂ (IIIa)

oder

Ar₂Bi(CₘF₂ₘ₊₁) (IIIb),

mit Chlorwasserstoff, Bromwasserstoff, wasserfreier HF oder Trifluormethansulfonsäure zu Verbindungen der Formel (I) umgesetzt werden, wobei Ar jeweils unabhängig voneinander eine Arylgruppe mit 6 bis 10 C-Atomen, die substituiert oder unsubstituiert sein kann, bedeutet und wobei die Bedingungen der Umsetzungen mit der Verbindung der Formel (II) derart gewählt werden, dass sowohl der Wassergehalt als auch der Sauerstoffgehalt maximal 100 ppm betragen.

Alternativ zu Verbindungen der Formel (II) können auch in dieser Reaktion Grignardreagenzien verwendet werden, die beispielsweise der Formel (IIA) entsprechen,

(CₘF₂ₘ₊₁)MgY (IIA),

wobei
m 2, 3 oder 4 bedeutet und Y I oder Br bedeutet. Die für die Umsetzung mit Verbindungen der Formel (II) genannten Reaktionsbedingungen, dass sowohl der Wassergehalt als auch der Sauerstoffgehalt maximal 100 ppm betragen soll, gelten entsprechend auch für die Umsetzung der Verbindungen der Formel (IIA).

Ein weiterer Gegenstand der Erfindung sind ebenfalls die Verbindungen der Formel (IIIa) und (IIIb)

ArBi(CₘF₂ₘ₊₁)₂ (IIIa),

Ar₂Bi(CₘF₂ₘ₊₁) (IIIb),

wobei
m jeweils unabhängig voneinander 2, 3 oder 4 bedeutet und
Ar jeweils unabhängig voneinander eine Arylgruppe mit 6 bis 10 C-Atomen bedeutet, die substituiert sein kann.

Eine Arylgruppe mit 6 bis 10 C-Atomen bedeutet Phenyl oder Naphthyl, das einfach oder mehrfach durch Alkyl, fluoriertes Alkyl, OAlkyl oder N(Alkyl)₂ substituiert sein kann.
"Alkyl" bedeutet eine lineare oder verzweigte Alkylgruppe mit 1 bis 10 C-Atomen.
"Fluoriertes Alkyl" ist eine lineare oder verzweigte fluorierte Alkylgruppe mit 1 bis 10 C-Atomen, wobei mindestens ein H-Atom einer linearen oder verzweigten Alkylgrupe mit 1 bis 10 C-Atomen durch ein F-Atom ersetzt ist. Es können auch alle H-Atome durch F-Atome ersetzt sein.

Bevorzugt ist die Arylgruppe unsubstituiertes oder einfach durch Alkyl, fluoriertes Alkyl, OAlkyl oder N(Alkyl)₂ substituiertes Phenyl. Besonders bevorzugt ist Ar eine unsubstituierte Phenylgruppe oder eine einfach mit Alkyl substituierte Phenylgruppe. Ganz besonders bevorzugt ist Ar eine unsubstituierte Phenylgruppe.

Eine geradkettige oder verzweigte Alkylgruppe mit 1 bis 10 C-Atomen ist beispielsweise Methyl, Ethyl, *iso*-Propyl, Propyl, Butyl, *sec*-Butyl oder *tert-*Butyl, Pentyl, 1-, 2- or 3-Methylbutyl, 1,1-, 1,2- or 2,2-Dimethylpropyl, 1-Ethylpropyl, *n*-Hexyl, *n*-Heptyl, *n*-Octyl, *n*-Nonyl oder *n*-Decyl.
Bevorzugt ist "fluoriertes Alkyl" eine geradkettige oder verzweigte fluorierte Alkylgruppe mit 1 bis 4 C-Atomen. Besonders bevorzugt ist "fluoriertes Alkyl" Trifluormethyl, Pentafluorethyl, Heptafluor-*iso*-Propyl, Heptafluorpropyl und Nonafluorbutyl.

Besonders bevorzugt ist die Verbindung der Formel (IIIa) Bis(pentafluorethyl)phenylbismutan.
Besonders bevorzugt ist die Verbindung der Formel (IIIb) Pentafluorethyldiphenylbismutan.

Die Verbindungen der Formel (IIIa) und (IIIb) sind ebenfalls als Lewis-Säure-Katalysatoren geeignet.

Ein weiterer Gegenstand der Erfindung ist daher die Verwendung mindestens einer Verbindung der Formel (IIIa) oder (IIIb), wie zuvor beschrieben oder bevorzugt beschrieben, als Lewis-Säure-Katalysator.

Ein weiterer Gegenstand der Erfindung ist daher ebenfalls ein Verfahren zur Herstellung von Verbindungen der Formel (IIIa) oder (IIIb), dadurch gekennzeichnet, dass eine Verbindung der Formel (II)

(CₘF₂ₘ₊₁)ₙLi (II),

wobei
m jeweils unabhängig voneinander 2, 3 oder 4 bedeutet, mit Dichlorarylbismutan oder Chlor(diaryl)bismutan umgesetzt wird, wobei Aryl im ensprechenden Bismutan eine Arylgruppe mit 6 bis 10 C-Atomen bedeutet, die substituiert oder unsubstituiert sein kann.

Die Verbindungen der Formel (II) können ebenfalls *in situ* hergestellt werden, wie zuvor beschrieben.

Dichlorphenylbismutan ist kommerziell erhältlich. Die Synthese von Dichlorphenylbismutan und Diphenylchlorbismutan ist beispielsweise in S. Faleschini et al, Journal of Organometallic Chemistry, 1972, 44, 317-323 oder in D.H.R. Barton et al, Tetrahedron, 1986, 42, 3111-3122 beschrieben.

Die Verbindungen Dichlorarylbismutan und Diarylchlorbismutan können analog zu diesen Verfahren hergestellt werden.

Die Umsetzung mit Dichlorarylbismutan oder Chlor(diaryl)bismutan, bevorzugt die Umsetzung von Dichlorarylbismutan, erfolgt direkt nach der *in situ* Herstellung der Verbindung der Formel (II), wie zuvor beschrieben, in dem vorhandenen Lösemittel oder Lösemittelgemisch zu den Verbindungen der Formel (IIIa) oder (IIIb).

Bevorzugt werden die Startmaterialien bei tiefer Temperatur, beispielsweise bei Temperaturen zwischen -100°C und -65°C gemischt. Dann wird das Gemisch bevorzugt auf eine Temperatur zwischen -10°C und 5°C erwärmt, vorzugsweise auf 0°C. Die Reaktionsmischung wird dann unter Inertgasatmosphäre filtriert.

Es schließt sich bevorzugt eine geeignete Aufreinigungsmethode zur Isolierung der Verbindungen der Formel (IIIa) oder (IIIb) an. Beispielsweise Waschen des Rückstands mit einem geeigneten Lösemittel und anschließender Sublimation oder Kristallisation.

Für die Umsetzung mit Chlorwasserstoff, Bromwasserstoff, wasserfreier HF oder Trifluormethansulfonsäure wird die isolierte Verbindung der Formel (IIIa) oder der Formel (IIIb) vorgelegt und vorzugsweise entgast. Danach wird Chlorwasserstoff, Bromwasserstoff, wasserfreie HF oder Trifluormethansulfonsäure aufkondensiert und die Reaktionsmischung vorzugsweise bei Temperaturen von 0°C bis 70°C, besonders bevorzugt bei einer Badtemperatur von 10°C bis 60°C, gerührt. Überschüssiger Chlorwasserstoff oder Bromwasserstoff oder überschüssige HF oder Trifluormethansulfonsäure wird durch Kondensation entfernt und der Rückstand wird vorzugsweise zur Aufreinigung sublimiert.

Verbindungen der Formel (I), wie zuvor beschrieben, in denen X F oder OSO₂CF₃ bedeutet, können alternativ aus entsprechenden Verbindungen der Formel (I), in denen X Cl bedeutet hergestellt werden, in dem mit AgF oder AgOSO₂CF₃ umgesetzt wird. Die Umsetzung erfolgt vorzugsweise in einem organischen Lösemittel oder in einer Mischung von organischen Lösemitteln. Besonders bevorzugt wird eine Mischung aus Dichlormethan und Acetonitril (1:1) eingesetzt. Die Reaktionstemperatur ist vorzugsweise Raumtemperatur. Weiterhin ist es bevorzugt, die Reaktion unter Inertgasbedingungen und unter Lichtausschluss durchzuführen.

Ein weiterer Gegenstand der Erfindung ist die Verwendung der Verbindungen der Formel (I) oder der Formeln (IIIa) und (IIIb), wie zuvor beschrieben oder als bevorzugt beschrieben zur Verwendung in einer Lewis-Säure-katalysierten Reaktion.
In einer bevorzugten Ausführungsform der Erfindung wird die Lewis-Säure-katalysierte Reaktion ausgewählt aus einer Kondensationsreaktion, Alkoholyse, Aldol-Reaktion, Mukaiyama-Aldol-Reaktion, Gattermann-Koch-Reaktion, Beckmann- und Fries-Umlagerung, Friedel-Crafts-Acylierung, Friedel-Crafts-Alkylierung, Mannich-Reaktion, Diels-Alder-Reaktion, Aza-Diels-Alder-Reaktion, Baylis-Hillman-Reaktion, Reformatskyreaktion, Claisen-Umlagerung, Cyclisierungsreaktion nach Prins, Allylierung von Carbonaylverbindungen, Cyanierung von Aldehyden und Ketonen, Cyanosilylierung von Aldehyden und Ketonen, 1,3-dipolare Cycloaddition oder Michael-Reaktion.

Die Verbindungen der Formel (I) werden bevorzugt in einer Katalysatormenge von 0,01 bis 10 mol% bezogen auf das Startmaterial im Unterschuss eingesetzt. Besonders bevorzugt werden die Verbindungen der Formel (I), wie zuvor beschrieben oder als bevorzugt beschrieben, in einer Menge von 1 bis 5 mol% eingesetzt. Der Fachmann auf dem Gebiet der Katalyse ist in der Lage, die optimale Katalysatormenge für die entsprechende zu katalysierende Reaktion auszuwählen. Die Ergebnisse im Beispielteil bestätigen dass (C₂F₅)BiCl₂ und (C₂F₅)₂BiCl deutlich aktivere Katalysatoren in Diels-Alder Reaktion sind, im Vergleich zu BiCl₃.

Aufgrund der spezifischen Lösungseigenschaften der Verbindungen der Formel (I), wie zuvor beschrieben oder als bevorzugt beschrieben, ist die Wahl des Lösemittels für die Lewis-Säure-katalysierte Reaktion entscheidend.

Geeignete protische Lösemittel bei Verwendung der erfindungsgemäßen Lewis-Säure-Katalysatoren sind Ethanol oder Methanol.

Geeignete aprotische Lösemittel bei Verwendung der erfindungsgemäßen Lewis-Säure-Katalysatoren sind Acetonitril, Propionitril, Benzonitril, Nitromethan, Diethylether, Methyl-*tert*-Butylether, 1,4-Dioxan, Tetrahydrofuran, 2-Methyltetrahydrofuran, Dichlormethan, 1,2-Dichlorethan, Monoglyme, Diglyme, Triglyme, Hexan, Heptan, Petrolether, Benzol oder Toluol.

Auch die Klasse der ionischen Flüssigkeiten sind als Lösemittel bei Verwendung der erfindungsgemäßen Lewis-Säure-Katalysatoren geeignet.

Unter einer ionischen Flüssigkeit versteht man Salze, die in der Regel aus einem organischen Kation und einem anorganischen Anion bestehen. Sie enthalten keine neutralen Moleküle und weisen meistens Schmelzpunkte kleiner 373 K auf [Wasserscheid P, Keim W, Angew. Chem. 112, 2000, 3926]. Ionische Flüssigkeiten haben bedingt durch Ihren Salzcharakter einzigartige Stoffeigenschaften, wie beispielsweise einen niedrigen Dampfdruck, einen flüssigen Zustand über einen breiten Temperaturbereich, sind nicht entflammbar, zeigen eine hohe elektrische Leitfähigkeit und eine hohe elektrochemische und thermische Stabilität.

Geeignete ionische Flüssigkeiten als Lösemittel bei Verwendung der erfindungsgemäßen Lewis-Säure-Katalysatoren sind ionische Flüssigkeiten, die ein organisches Kation haben und deren Anion ausgewählt wird aus der Gruppe [R₁SO₃]⁻, [R₂COO]⁻, [R₂SO₃]⁻, [R₁OSO₃]⁻, [BF₄]⁻, [HSO₄]¹⁻, [(R₁)₂P(O)O]⁻, [(R₂)₂P(O)O]⁻, [R₂P(O)O₂]²⁻, [(FSO₂)₂N]⁻, [(R₂SO₂)₂N]⁻, [(R₂SO₂)₃C]⁻, [(FSO₂)₃C]⁻, [P(R₂)_{y}F_{6-y}]⁻, [BFₓ(R₂)₄₋ₓ]⁻, [BFₓ(CN)₄₋ₓ]⁻, [B(R₁)ₐ(CN)₄₋ₐ]⁻, [B(R₂)F₂(CN)]⁻ oder [B(R₂)F(CN)₂]⁻,
wobei R₁ jeweils unabhängig voneinander eine lineare oder verzweigte Alkylgruppe mit 1 bis 12 C-Atomen bedeutet,
R₂ jeweils unabhängig voneinander eine teilfluorierte oder perfluorierte lineare oder verzweigte Alkylgruppe mit 1 bis 12 C-Atomen bedeutet oder Pentafluorphenyl,
x die ganze Zahl 0, 1, 2 oder 3 bedeutet,
y die ganze Zahl 0, 1, 2, 3 oder 4 bedeutet und
a die ganze Zahl 1 oder 2 bedeutet.

Eine perfluorierte lineare oder verzweigte Alkylgruppe mit 1 bis 4 C-Atomen ist beispielsweise Trifluormethyl, Pentafluorethyl, *n*-Heptafluorpropyl, *iso-*Heptafluorpropyl, *n*-Nonafluorbutyl, *sec*-Nonafluorbutyl oder *tert-*Nonafluorbutyl. R₂ definiert in Analogie eine lineare oder verzweigte perfluorierte Alkylgruppe mit 1 bis 12 C-Atomen, umfassend die zuvor genannten Perfluoralkylgruppen und beispielsweise perfluoriertes n-Hexyl, perfluoriertes *n*-Heptyl, perfluoriertes *n*-Octyl, perfluoriertes Ethylhexyl, perfluoriertes *n*-Nonyl, perfluoriertes *n*-Decyl, perfluoriertes *n*-Undecyl oder perfluoriertes *n*-Dodecyl.

Bevorzugt ist R₂ Trifluormethyl, Pentafluorethyl oder Nonafluorbutyl, ganz besonders bevorzugt Trifluormethyl oder Pentafluorethyl.

Die Variable y ist bevorzugt 1, 2 oder 3, besonders bevorzugt 3.

Bevorzugte Lösemittel sind ionische Flüssigkeiten mit den Anionen [P(R₂)_{y}F_{6-y}]⁻ und [R₂SO₃]⁻, wobei R₂ und y eine zuvor angegebene oder als bevorzugt angegebene Bedeutung haben.

Besonders bevorzugte Lösemittel sind ionische Flüssigkeiten mit den Anionen [P(C₂F₅)₃F₃]⁻ und [CF₃SO₃]⁻.

Die organischen Kationen sind in der Regel nicht eingeschränkt und werden bevorzugt aus Imidazoliumkationen, Pyridiniumkationen oder Pyrrolidinium-Kationen ausgewählt, die entsprechend substituiert sein können, wie aus dem Stand der Technik bekannt.

Ganz besonders bevorzugt wird die ionische Flüssigkeit 1-Ethyl-3-methylimidazolium-tris(pentafluorethyl)trifluorphosphat {[EMIM][FAP]} als Lösemittel ausgewählt.

Die folgenden Beispiele von Lewis-Säure-katalysierten Reaktionen zeigen, dass durch den Einsatz der Verbindungen der Formel (I), wie zuvor beschrieben oder als bevorzugt beschrieben, im direkten Vergleich mit BiCl₃ ebenfalls sehr gute Ausbeuten erzielt werden können, wobei jedoch die Menge an Katalysator bei den erfindungsgemäßen Lewis-Säure-Katalysatoren signifikant reduziert ist.

Die Eignung der Verbindungen der Formel (I) als Lewis-Säure-Katalysatoren ist anhand einer Diels-Alder-Reaktion und einer Cyanosilylierung eines Aldehyds belegt. Diese Reaktionstypen sind repräsentativ für Lewis-Säure-katalysierte Reaktionen.

Auch ohne weitere Ausführungen wird davon ausgegangen, dass ein Fachmann die folgenden Beschreibungen im weitesten Umfang nutzen kann. Die bevorzugten Ausführungsformen und Beispiele sind deswegen lediglich als beschreibende, keineswegs als in irgendeiner Weise limitierende Offenbarung aufzufassen.

### Beispiele:

### Beispiel 1: Synthese von Tris(pentafluorethyl)bismutan, Bi(C₂F₅)₃.

### A)

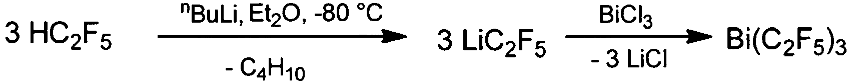

In einem 500 mL-Schlenkkolben werden im Stickstoffgegenstrom 50 mL (100 mmol) einer 2 M Lösung von n-Buthyllithium in Pentan in 200 mL trockenem Diethylether vorgelegt und bei -90 °C entgast. Es werden 12.3 g (102.5 mmol) Pentafluorethan bei -80 °C aufkondensiert und 30 Minuten bei gleicher Temperatur gerührt. Im Stickstoffgegenstrom werden 6.31 g (20.0 mmol) Bismuttrichlorid, BiCl₃, hinzugegeben. Das Reaktionsgemisch wird für 60 Minuten bei -80 °C gerührt und danach innerhalb 4 Stunden auf 0 °C erwärmt. Die Reaktionsmischung wird unter Inertgasatmosphäre filtriert und der Rückstand zweimal mit je 20 mL trockenem n-Pentan gewaschen. Im statischen Vakuum (70mbar) wird die Reaktionsmischung eingeengt. In einer Sublimationsapparatur wird im Hochvakuum das verbleibende Lösemittel entfernt und das Reaktionsprodukt an einem mit Trockeneis gekühlten Kühlfinger (-50 ° C bis -30 ° C) sublimiert. Nach Erwärmung auf Raumtemperatur werden 7.16 g Tris(pentafluorethyl)bismutan, Bi(C₂F₅)₃, als eine farblose Flüssigkeit erhalten. Ausbeute (bezogen auf Bismuttrichlorid): 63% (12.6 mmol). Schmelzpunkt: ∼ -10 °C.
Die NMR-Daten entsprechen den aus der Literatur bekannten Werten [D. Naumann, W. Tyrra, J. Organomet. Chem. 1987, vol. 334, pp. 323-328].

**Tabelle: NMR-Daten von Tris(pentafluorethyl)bismutan, Bi(C₂F₅)₃, in CH₃CN**

| Kern | δ [ppm] | Aufspaltung | J [Hz] | Zuordnung |
|---|---|---|---|---|
| ¹⁹F | -82.1 | s | - | CF₃CF₂ |
| | -100.1 | s | - | CF₃CF₂ |
| ¹³C, ¹⁹F-DEPT 45nd | 122.6 | qt | ¹*J*_{CF} = 285 | CF₃CF₂ |
| | 160.9 | m | | CF₃CF₂ |

**Tabelle: Massenspektrometrische Daten von Tris(pentafluorethyl)bismutan, Bi(C₂F₅)₃, EI-TOF (Gaseinlass), positiv, 70 eV.**

| m/z | Rel. Intensität (%) | Fragment |
|---|---|---|
| 466.9 | 50 | [BiF(C₂F₅)₂]⁺ |
| 346.9 | 100 | [BiF[(C₂F₅)]⁺ |

### B)

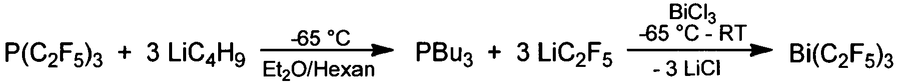

Eine Lösung von 171 mg (0.44 mmol) P(C₂F₅)₃ in Et₂O (10 ml) wird bei -65 °C mit einer 1.6 M Lösung von *n*-Butyllithium in Hexan (0.80 ml, 1.28 mmol) versetzt und 10 Minuten gerührt. Bei gleicher Temperatur werden 0.15 g (0.48 mmol) BiCl₃ zugegeben und das Reaktionsgemisch langsam auf Raumtemperatur erwärmt. Die Lösung wird NMR-spektroskopisch untersucht. Die NMR-Daten bestätigen die Bildung von Bi(C₂F₅)₃.

### Beispiel 2: Darstellung von Bis(pentafluorethyl)phenylbismutan, PhBi(C₂F₅)₂.

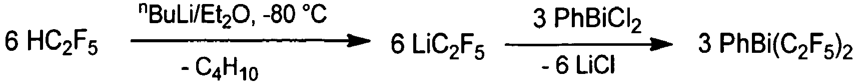

In einem 250 mL-Schlenkkolben werden 22 mL (44.0 mmol) einer 2 M Lösung von *n*-Buthyllithium in Pentan in 100 mL Et₂O vorgelegt und bei -80 °C entgast. Es werden 5.40 g (45 mmol) Pentafluorethan bei -80 °C aufkondensiert und für 25 Minuten bei gleicher Temperatur gerührt. Es werden 3.87 g (10.8 mmol) Dichlorphenylbismutan zugegeben und für 3.5 Stunden im Kältebad -80 °C gerührt. Die Suspension wird in Inertgasatmosphäre filtriert und im statischen Hochvakuum eingeengt. In einer Sublimationsapparatur wird im Hochvakuum und bei einer Ölbadtemperatur von 80 °C das verbleibende Lösemittel entfernt und das Reaktionsprodukt an einem mit Trockeneis gekühlten Kühlfinger (coldfinger) (-35 °C) sublimiert. Nach Erwärmung auf Raumtemperatur werden 4.22 g Bis(pentafluorethyl)phenylbismutan, PhBi(C₂F₅)₂, als farblose Flüssigkeit erhalten.
Ausbeute (bezogen auf Dichlorphenylbismutan): 8.1 mmol, 75 %. Schmelzpunkt: - 15 °C.

Das Produkt wird mittels NMR- und IR-Spektroskopie, sowie über die Massenspektrometrie charakterisiert.

**Tabelle: NMR-Daten von Bis(pentafluorethyl)phenylbismutan, PhBi(C₂F₅)₂, in CD₃CN.**

| Kern | δ [ppm] | Aufspaltung | J [Hz] | Zuordnung |
|---|---|---|---|---|
| ¹H | 8.2 | d | ³*J*_{HH} = 7 | ortho H |
| | 7.7 | t | ³*J*_{HH} = 7 | meta H |
| | 7.5 | t | ³*J*_{HH} = 7 | para H |
| ¹⁹F | -82.4 | m | - | (CF₃CF₂)₂ BiPh |
| | -105.8 | s | - | (CF₃CF₂)₂ BiPh |
| ¹³C-CPD; | 170.4 | s | | quart. C |
| ¹H, ¹³C- | 138.7 | s | | ortho C |
| HMBC | 132.0 | s | | meta C |
| | 130.1 | s | | para C |
| ¹³C, ¹⁹F-DEPT45; | 122.3 | qm | ¹*J*_{CF} = 283 | CF₃CF₂ |
| ¹³C, ¹⁹F-HMBC | 148.3 | tm | | CF₃CF₂ |

**Tabelle: Massenspektrometrische Daten von Bis(pentafluorethyl)phenyl-bismutan, PhBi(C₂F₅)₂, EI-TOF (Gaseinlass), positiv, 70 eV.**

| m/z | rel. Intensität (%) | Fragment |
|---|---|---|
| 405.0 | 56 | [(C2F5)BiPh]+ |
| 286.1 | 62 | [PhBi]+ |
| 209.0 | 91 | Bi+ · |

**IR: *ṽ*** = 3065 (w), 2965 (w), 1303 (m), 1182 (s), 1070 (s), 896 (s), 726 (s), 691 (m), 600 (w) 533 (w), 442 (w)

### Beispiel 3: Darstellung von Bis(pentafluorethyl)chlorbismutan, (C₂F₅)₂BiCl.

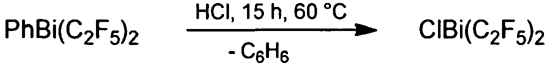

In einer 800 mL-Younghahnampulle werden 4.02 g (7.7 mmol) Bis(pentafluorethyl)-phenylbismutan, PhBi(C₂F₅)₂, vorgelegt und entgast. Es werden 1.09 g (30.0 mmol) Chlorwasserstoff aufkondensiert und bei 60 °C Ölbadtemperatur für 2.5 h gerührt. Überschüssiger Chlorwasserstoff wird durch Kondensation entfernt und der Rückstand bei 90 °C im Hochvakuum sublimiert. Bis(pentafluorethyl)chlorbismutan, (C₂F₃)2BiCl, wird als gelber Feststoff erhalten. Ausbeute (bezogen auf Bis(pentafluorethyl)phenylbismutan): 3.45 g (7.2 mmol, 92 %). Schmelzpunkt: 75 - 76 °C. Das Produkt wird mittels NMR- und IR-Spektroskopie, sowie über die Massenspektrometrie charakterisiert.

**Tabelle: NMR-Daten von Bis(pentafluorethyl)chlorbismutan, (C₂F₅)₂BiCl, in CH₃CN.**

| Kern | δ [ppm] | Aufspaltung | J [Hz] | Zuordnung |
|---|---|---|---|---|
| 19F | -80.6 | s | - | **CF₃**CF₂ |
| | -103.0 | s | - | CF₃**CF₂** |

**Tabelle: NMR-Daten von Bis(pentafluorethyl)chlorbismutan, (C₂F₅)₂BiCl, in CH₂Cl₂.**

| Kern | δ [ppm] | Aufspaltung | J [Hz] | Zuordnung |
|---|---|---|---|---|
| 19F | -80.8 | s | - | CF₃CF₂ |
| | -101.7 | AA'BB' | - | CF₃CF₂ |

**Tabelle: Massenspektrometrische Daten von Bis(pentafluorethyl)-chlorbismutan, (C₂F₅)₂BiCl, EI-TOF (Gaseinlass), positiv, 70 eV.**

| m/z | rel. Intensität (%) | Fragment |
|---|---|---|
| 467.1 | 20 | [BiF(C₂F₅)₂]⁺ |
| 447.1 | 33 | [Bi(C₂F₅)₂]⁺ |
| 347.1 | 48 | [BiF[(C₂F₅)]⁺ |
| 328.1 | 6 | [Bi(C₂F₅)]⁺ |
| 247.1 | 10 | [BiF₂]⁺ |
| 209.0 | 59 | Bi⁺ · |

**IR: *ṽ* =** 1301 (m), 1190 (s), 1129 (m), 1082 (s), 895 (s), 730 (m), 600 (m), 534 (w).

### Beispiel 4: Darstellung von Pentafluorethyldiphenylbismutan, Ph₂BiC₂F₅.

### A)

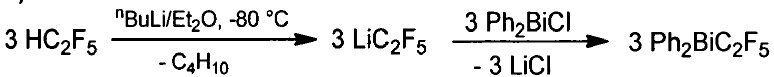

In einem 500 mL-Schlenkkolben werden 20 mL (40 mmol, 2 M in n-Pentan) n-BuLi in 150 mL Diethylether vorgelegt und bei -80 °C entgast. Es werden 5.4 g (45 mmol) Pentafluorethan aufkondensiert und für 10 min bei gleicher Temperatur gerührt. Es werden 7.97 g (20 mmol) Chlor(diphenyl)bismutan zugegeben, für 4 h bei -60 °C gerührt und anschließend auf Raumtemperatur erwärmt. Alle flüchtigen Bestandteile werden im Hochvakuum entfernt, der erhaltene Rückstand in n-Pentan aufgenommen, in Inertgasatmosphäre filtriert und mit n-Pentan gewaschen. Nach Entfernen des Lösemittels wird Pentafluorethyl(diphenyl)bismutan, Ph₂BiC₂F₅, in Form farbloser Kristalle erhalten, welche sich nach kurzer Zeit gelb-braun färben. Ausbeute (bezogen auf Chlor(diphenyl)bismutan): 6.20 g (12.9 mmol, 65 %).

**IR: *ṽ* =** 3052 (w), 2957 (w), 1303 (m), 1568 (w), 1475 (w), 1428 (w), 1308 (m), 1262 (w), 1176 (s), 1075 (s), 903 (m), 800 (w), 724 (s), 692 (s), 596 (m) 533 (w), 442 (m)

**Tabelle: NMR-Daten von Pentafluorethyldiphenylbismutan in CD₂Cl₂.**

| Kern | δ [ppm] | Aufspaltung | J [Hz] | Zuordnung |
|---|---|---|---|---|
| ¹H | 7.9 | d | ³*J*_{HH} = 7.2 | ortho H |
| | 7.6 | t | ³*J*_{HH} = 7.4 | meta H |
| | 7.4 | t | ³*J*_{HH} = 7.4 | para H |
| ¹⁹F | -81.6 | s | - | CF₃CF₂ |
| | -105.6 | AA'BB' | - | CF₃CF₂ |
| ¹³C-CPD | 160.5 | s | | quart. C |
| ¹H, ¹³C-HMBC | 137.8 | s | | ortho C |
| | 131.1 | s | | meta C |
| | 128.9 | s | | para C |
| ¹³C, ¹⁹F- | 201.5 | s | | CF₃CF₂ |
| DEPT45;¹³C, ¹⁹F-HMBC | 129.4 | s | | CF₃CF₂ |

### B)

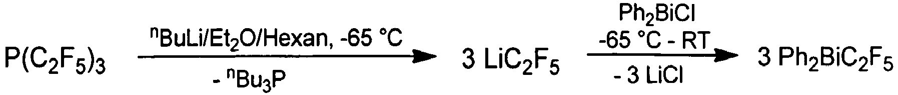

In einem 250 mL-Schlenkkolben werden 80 mL Diethylether vorgelegt und bei -196 °C entgast. Es werden 2.95 g (7.6 mmol) Tris(pentafluorethyl)phosphan, (C₂F₅)₃P, dazu aufkondensiert und auf -65 °C gebracht. Zu diesem Gemisch werden 9.43 g (22.2 mmol; 13.9 mL) einer 1.6 M Lösung von n-BuLi in Hexan zugegeben und bei gleicher Temperatur für 15 min gerührt. Zu der Reaktionsmischung werden 8.75 g (22.0 mmol) Chlor(diphenyl)bismutan zugegeben und für 3 h unter Rühren bis 0 °C erwärmt. Die erhaltene Suspension wird unter Inertgasatmosphäre filtriert, der Rückstand mit wenig Diethylether gewaschen und alle flüchtigen Bestandteile des Filtrats werden im Hochvakuum entfernt. Der erhaltene Rückstand wird in 100 mL *n*-Pentan aufgenommen. Die Lösung wird bis zur ersten Kristallbildung eingeengt und das Produkt durch Kühlung auf -28 °C auskristallisiert. Die Kristalle werden mit wenig *n*-Pentan gewaschen und in Hochvakuum getrocknet.
Pentafluorethyl(diphenyl)bismutan, Ph₂BiC₂F₅, wird in Form farbloser, hydrolyse-empfindlicher Kristalle erhalten. Ausbeute (bezogen auf Chlor(diphenyl)bismutan): 8.57 g (17.8 mmol, 81 %).
Das Produkt wird mittels NMR- und IR-Spektroskopie charakterisiert.. Die NMR- und IR-Daten entsprechen den in Beispiel 4A) gegebenen Werten.

### Beispiel 5. Darstellung von Pentafluorethyldichlorbismutan, (C₂F₅)BiCl₂

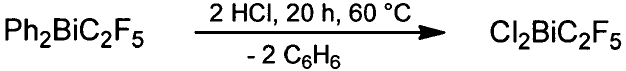

6.20 g (12.9 mmol) Diphenyl(pentafluorethyl)bismutan, Ph₂BiC₂F₅, werden in 25 mL Diethylether aufgenommen und in eine 800 mL-Younghahnampulle überführt. Das Lösemittel wird im Hochvakuum entfernt und es werden 1.46 g (40 mmol) Chlorwasserstoff aufkondensiert. Das Reaktionsgemisch wird bei 60 °C Ölbadtemperatur für 20 h gerührt. Alle flüchtigen Bestandteile werden durch Kondensation entfernt, der Rückstand in Dichlormethan aufgenommen und in einen 100 mL-Schlenkkolben überführt. Durch Sublimation im Hochvakuum bei 90 °C Ölbadtemperatur und einer Kühlfingertemperatur von -78 °C wird
Pentafluorethyldichlorbismutan, C₂F₅BiCl₂, als gelber Feststoff erhalten. Ausbeute (bezogen auf Pentafluorethyl(diphenyl)bismutan): 4.67 g (11.7 mmol, 91 %).
Schmelzpunkt: 104 °C

Das Produkt wird mittels NMR-, und IR-Spektrometrie charakterisiert.

**Tabelle: NMR-Daten von Pentafluorethyldichlorbismutan, C₂F₅BiCl₂, in CH₃CN.**

| Kern | δ [ppm] | Aufspaltung | J [Hz] | Zuordnung |
|---|---|---|---|---|
| 19F | -79.5 | s | ¹*J*_{CF} = 283 | C**F₃**CF₂ |
| | | | ²*J*_{CF} = 44 | |
| | -105.2 | s | ¹*J*_{CF} = 333 | CF₃C**F₂** |
| ¹⁹F, ¹³C-HMBC | 129.42 | | ¹*J*_{CF} = 283 | **C**F₃CF₂ |
| | | | ²*J*_{CF} = 44 | |
| | 201.45 | | ¹*J*_{CF} = 333 | CF₃**C**F₂ |
| | | | ²*J*_{CF} = 27 | |

**IR:** *ṽ* **=** 1301 (m), 1267 (w), 1103 (s), 1073 (s), 897 (s), 729 (s), 603 (m), 584 (w), 531 (w).

### Beispiel 6. Darstellung von Bis(pentafluorethyl)fluorbismutan

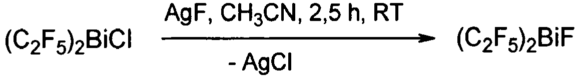

Zu einer Lösung von (C₂F₅)₂BiCl (1.38 g, 2.86 mmol) in einer Mischung aus Dichlormethan (10 mL) und Acetonitril (10 mL) werden im Stickstoffgegenstrom 366 mg (2.885 mmol) Silberfluorid gegeben. Die Reaktionsmischung wird unter Lichtausschluss für 2.5 h bei Raumtemperatur gerührt. Der ausgefallene Feststoff wird unter Inertgasatmosphäre abfiltriert und das Filtrat im Hochvakuum eingeengt. Nach dem Trocknen im Hochvakuum wird (C₂F₅)₂BiF als farbloser, leicht kristalliner Feststoff erhalten. Ausbeute ist 1.13 g (2.42 mmol, 85 % bezogen auf (C₂F₅)₂BiCl).
Zersetzungspunkt: >200 °C.

**Tabelle: NMR-Daten von (C₂F₅)₂BiF, in CD₃CN**

| Kern | δ [ppm] | Aufspaltung | J [Hz] | Zuordnung |
|---|---|---|---|---|
| ¹⁹F | -81.8 | s | ¹*J*CF = 282 | C**F**3CF2 |
| | -108.4 | s | | CF3C**F**2 |
| | -171.9 | s | | Bi**F** |
| ¹³C, ¹⁹F-DEPT4 5; | 125.7 | s | ¹*J*CF = 282 | **C**F3CF2 |
| ¹³C, ¹⁹F-HMBC | 175.9 | - | | CF3**C**F2 |

IR (Feststoff): *ṽ* = 429 (vw), 535 (vw), 588 (vw), 602 (w), 732 (m), 899 (s), 1079 (s), 1186 (s), 1303 (m), 1648 (vw) cm⁻¹.

### Beispiel 7. Bismut(III) katalysierte Diels-Alder-Reaktion

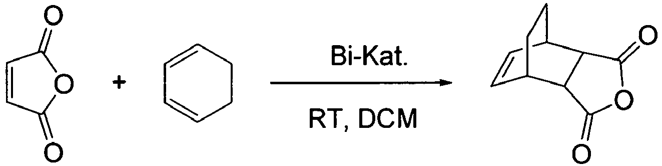

Die Diels-Alder-Reaktion von Maleinsäureanhydrid und 1,3-Cyclohexadien zu 3',4,7,7'-Tetrahydro-4,7-ethanisobenzofuran-1,3-dion wird bei RT in Dichlormethan als Lösemittel durchgeführt. Die Lösung wird schnell zitronengelb, wobei die Färbung im Laufe der Reaktion schwächer wird. In der folgenden Tabellesind die Reaktionsbedingungen, Einwaagen und Volumina der Edukte bzw. Katalysatoren angegeben.

**Tabelle: Reaktionsbedingungen, Einwaagen, Volumina und Umsätze der Diels-Alder-Reaktion.**

| Kat | Kat-Menge, mol % | Maleinsäureanhydrid, g (mmol), 1 Äq. | 1,3-Cyclohexadien, mL (mmol), 1.5 Äq. | Zeit, min | Umsatz %* |
|---|---|---|---|---|---|
| BiCl₃ | 5 | 0.84 (8.6) | 1.2 (12.6) | 20 | 97 |
| (C₂F₅)BiCl₂ | 1 | 0.74 (7.5) | 1.1 (11.5) | 30 | 97 |
| (C₂F₅)₂BiCl | 1 | 0.42 (4.3) | 0.6 (6.3) | 30 | 89 |

| | | | | | |
|---|---|---|---|---|---|
| * Umsatzberechnungen beruhen auf ¹H- und ¹³C -NMR-spektroskopischen Messungen bezogen auf Maleinsäureanhydrid. | | | | | |

Die Ergebnisse bestätigen dass (C₂F₅)BiCl₂ und (C₂F₅)₂BiCl im Vergleich zu BiCl₃ deutlich aktivere Katalysatoren in Diels-Alder Reaktion sind.

### Beispiel 8. Cyanosilylierung katalysierte mit Tris(pentafluorethyl)bismutan, Bi(C₂F₅)₃.

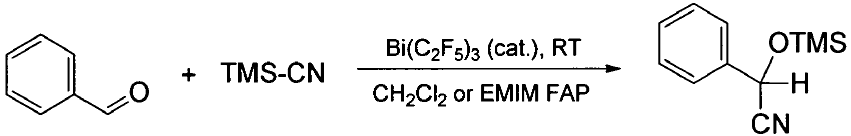

Die Cyanosilylierung von Benzaldehyd mit Trimethylsilylcyanid zu 2-Phenyl-2-(trimethylsilloxy)-acetonitril wird bei Raumtemperatur in CH₂Cl₂ or 1-Ethyl-3-Methylimidazolium Tris(pentafluorethyl)trifluorphosphat (EMIM FAP) als Lösemittel durchgeführt. In Tabelleunten sind die Reaktionsbedingungen, Einwaagen und Volumina der Edukte bzw. Katalysatoren angegeben.

**Tabelle: Reaktionsbedingungen, Einwaagen, Volumina und Umsätze der Cyanosilylierung.**

| Kat | Kat-Menge, mol %, (Lösemittel) | Benzaldehyd, mL (mmol) | Trimethylsilylcyanid, mL (mmol) | Zeit, h | Umsatz, %* |
|---|---|---|---|---|---|
| (C₂F₅)₃Bi | 5 (CH₂Cl₂) | 0.2 (2.0) | 0.4 (3.2) | 63 | 44 |
| | 5 (EMIM FAP) | 0.2 (0.2) | 0.3 (2.4) | 19 | 66 |

| | | | | | |
|---|---|---|---|---|---|
| * Umsatzberechnungen beruhen auf ¹H- und ¹³C -NMR-spektroskopischen Messungen bezogen auf Benzaldehyd. | | | | | |

Die Ergebnisse zeigen, dass durch die gute Löslichkeit von (C₂F₅)₃Bi in der hydrophoben ionischen Flüssigkeit die katalytische Aktivität von (C₂F₅)₃Bi in EMIM FAP im Vergleich zu üblichen organischen Lösemitteln, wie CH₂Cl₂, deutlich erhöht ist.

## Patentansprüche

1. Verwendung von mindestens einer Verbindung der Formel (I)
(CmF₂ₘ₊₁)ₙBiX₃₋ₙ (I),
wobei
m jeweils unabhängig voneinander 2, 3 oder 4 bedeutet;
n 1, 2 oder 3 bedeutet und
X F, Cl, Br oder OSO₂CF₃ bedeutet
als Lewis-Säure-Katalysator.

2. Verwendung nach Anspruch 1, wobei die Perfluoralkylgruppe (CₘF₂ₘ₊₁) in Formel (I) bei jedem Auftreten gleich ist.

3. Verwendung nach Anspruch 1 oder 2, wobei m 2 oder 4 bedeutet.

4. Verwendung nach einem oder mehreren der Ansprüche 1 bis 3, wobei X Cl bedeutet.

5. Verbindungen der Formel (I)
(CmF₂ₘ₊₁)ₙBiX₃₋ₙ (I),
wobei
m jeweils unabhängig voneinander 2, 3 oder 4 bedeutet;
n 1 oder 2 bedeutet und
X F, Cl, Br oder OSO₂CF₃ bedeutet.

6. Verbindungen nach Anspruch 5, **dadurch gekennzeichnet, dass** X Cl bedeutet.

7. Verfahren zur Herstellung von Verbindungen der Formel (I), wie in einem oder mehreren der Ansprüche 1 bis 3 beschrieben, wobei n 3 bedeutet, **dadurch gekennzeichnet, dass** eine Verbindung der Formel (II)
(CₘF₂ₘ₊₁)Li (II),
wobei
m 2, 3 oder 4 bedeutet, mit Bismuttrichlorid, Bismuttribromid oder Bismuttristriflat umgesetzt wird, wobei die Bedingungen der Umsetzungen derart gewählt werden, dass sowohl der Wassergehalt als auch der Sauerstoffgehalt maximal 100 ppm betragen.

8. Verfahren zur Herstellung von Verbindungen nach Anspruch 5 oder 6, **dadurch gekennzeichnet, dass** eine Verbindung der Formel (II)
(CₘF₂ₘ₊₁)Li (II),
wobei
m jeweils unabhängig voneinander 2, 3 oder 4 bedeutet, in einer ersten Umsetzung mit Dichlorarylbismutan oder Chlor(diaryl)bismutan umgesetzt wird, wobei Aryl im ensprechenden Bismutan eine Arylgruppe mit 6 bis 10 C-Atomen bedeutet, die substituiert oder unsubstituiert sein kann,
und nachfolgend die Zwischenprodukte der Formel (IIIa) oder (IIIb)
ArBi(CₘF₂ₘ₊₁)₂ (IIIa)
oder
Ar₂Bi(CₘF₂ₘ₊₁) (IIIb),
mit Chlorwasserstoff, Bromwasserstoff, wasserfreier HF oder CF₃SO₃H zu Verbindungen der Formel (I) umgesetzt werden, wobei Ar jeweils unabhängig voneinander eine Arylgruppe mit 6 bis 10 C-Atomen, die substituiert oder unsubstituiert sein kann, bedeutet und wobei die Bedingungen der Umsetzungen mit der Verbindung der Formel (II) derart gewählt werden, dass sowohl der Wassergehalt als auch der Sauerstoffgehalt maximal 100 ppm betragen.

9. Verwendung nach einem oder mehreren der Ansprüche 1 bis 4 in einer Lewis-Säure-katalysierten Reaktion ausgewählt aus einer Kondensationsreaktion, Alkoholyse, Aldol-Reaktion, Mukaiyama-Aldol-Reaktion, Gattermann-Koch-Reaktion, Beckmann- und Fries-Umlagerung, Friedel-Crafts-Acylierung, Friedel-Crafts-Alkylierung, Mannich-Reaktion, Diels-Alder-Reaktion, Aza-Diels-Alder-Reaktion, Baylis-Hillman-Reaktion, Reformatskyreaktion, Claisen-Umlagerung, Cyclisierungsreaktion nach Prins, Allylierung von Carbonaylverbindungen, Cyanierung von Aldehyden und Ketonen, Cyanosilylierung von Aldehyden und Ketonen, 1,3-dipolare Cycloaddition oder Michael-Reaktion.

10. Verbindungen der Formel (IIIa) und (IIIb)
ArBi(CₘF₂ₘ₊₁)₂ (IIIa),
Ar₂Bi(CₘF₂ₘ₊₁) (IIIb),
wobei
m jeweils unabhängig voneinander 2, 3 oder 4 bedeutet und
Ar jeweils unabhängig voneinander eine Arylgruppe mit 6 bis 10 C-Atomen bedeutet, die substituiert sein kann.

11. Verwendung von mindestens einer Verbindung nach Anspruch 10 als Lewis-Säure-Katalysator.

## Claims

1. Use of at least one compound of the formula (I)
(CmF₂ₘ₊₁)ₙBiX₃₋ₙ (I),
where
m in each case, independently of one another, denotes 2, 3 or 4;
n denotes 1, 2 or 3 and
X denotes F, Cl, Br or OSO₂CF₃,
as Lewis acid catalyst.

2. Use according to Claim 1, where the perfluoroalkyl group (CₘF₂ₘ₊₁) in formula (I) is the same on each occurrence.

3. Use according to Claim 1 or 2, where m denotes 2 or 4.

4. Use according to one or more of Claims 1 to 3, where X denotes Cl.

5. Compounds of the formula (I)
(CₘF₂ₘ₊₁)ₙBiX₃₋ₙ (I),
where
m in each case, independently of one another, denotes 2, 3 or 4;
n denotes 1 or 2 and
X denotes F, Cl, Br or OSO₂CF₃.

6. Compounds according to Claim 5, **characterised in that** X denotes Cl.

7. Process for the preparation of compounds of the formula (I), as described in one or more of Claims 1 to 3, where n denotes 3, **characterised in that** a compound of the formula (II)
(CₘF₂ₘ₊₁)Li (II),
where
m denotes 2, 3 or 4, is reacted with bismuth trichloride, bismuth tribromide or bismuth tristriflate, where the conditions of the reactions are selected in such a way that both the water content and also the oxygen content are a maximum of 100 ppm.

8. Process for the preparation of compounds according to Claim 5 or 6, **characterised in that** a compound of the formula (II)
(CₘF₂ₘ₊₁)Li (II),
where
m in each case, independently of one another, denotes 2, 3 or 4, is in a first reaction reacted with dichloroarylbismuthane or chloro(diaryl)bismuthane, where aryl in the corresponding bismuthane denotes an aryl group having 6 to 10 C atoms, which may be substituted or unsubstituted,
and the intermediates of the formula (IIIa) or (IIIb)
ArBi(CₘF₂ₘ₊₁)₂ (IIIa)
or
Ar₂Bi(CₘF₂ₘ₊₁) (IIIb),
are subsequently reacted with hydrogen chloride, hydrogen bromide, anhydrous HF or CF₃SO₃H to give compounds of the formula (I), where Ar in each case, independently of one another, denotes an aryl group having 6 to 10 C atoms, which may be substituted or unsubstituted, and where the conditions of the reactions with the compound of the formula (II) are selected in such a way that both the water content and also the oxygen content are a maximum of 100 ppm.

9. Use according to one or more of Claims 1 to 4 in a Lewis acid-catalysed reaction selected from a condensation reaction, alcoholysis, aldol reaction, Mukaiyama aldol reaction, Gattermann-Koch reaction, Beckmann and Fries rearrangement, Friedel-Crafts acylation, Friedel-Crafts alkylation, Mannich reaction, Diels-Alder reaction, aza Diels-Alder reaction, Baylis-Hillman reaction, Reformatsky reaction, Claisen rearrangement, Prins cyclisation reaction, allylation of carbonyl compounds, cyanation of aldehydes and ketones, cyanosilylation of aldehydes and ketones, 1,3-dipolar cycloaddition or Michael reaction.

10. Compounds of the formulae (IIIa) and (IIIb)
ArBi(CₘF₂ₘ₊₁)₂ (IIIa),
Ar₂Bi(CₘF₂ₘ₊₁) (IIIb),
where
m in each case, independently of one another, denotes 2, 3 or 4 and
Ar in each case, independently of one another, denotes an aryl group having 6 to 10 C atoms, which may be substituted.

11. Use of at least one compound according to Claim 10 as Lewis acid catalyst.

## Revendications

1. Utilisation d'au moins un composé de formule (I)
(CₘF₂ₘ₊₁)ₙBiX₃₋ₙ (I),
où
m dans chaque cas, indépendamment les uns des autres, désigne 2, 3 ou 4 ;
n désigne 1, 2 ou 3 et
X désigne F, Cl, Br ou OSO₂CF₃,
comme catalyseur d'acide de Lewis.

2. Utilisation selon la revendication 1, dans laquelle le groupement perfluoroalkyle (CₘF₂ₘ₊₁) dans la formule (I) est le même à chaque occurrence.

3. Utilisation selon la revendication 1 ou 2, dans laquelle m désigne 2 ou 4.

4. Utilisation selon l'une ou plusieurs parmi les revendications 1 à 3, dans laquelle X désigne Cl.

5. Composés de formule (I)
(CₘF₂ₘ₊₁)ₙBiX₃₋ₙ (I),
où
m dans chaque cas, indépendamment les uns des autres, désigne 2, 3 ou 4 ;
n désigne 1 ou 2 et
X désigne F, Cl, Br ou OSO₂CF₃.

6. Composés selon la revendication 5, **caractérisés en ce que** X désigne Cl.

7. Procédé de préparation de composés de formule (I), tels que décrits selon l'une ou plusieurs parmi les revendications 1 à 3, où n désigne 3, **caractérisé en ce qu'**un composé de formule (II)
(CₘF₂ₘ₊₁)Li (II),
où
m désigne 2, 3 ou 4, est réagi avec du trichlorure de bismuth, du tri-bromure de bismuth ou du tris(triflate) de bismuth, où les conditions des réactions sont sélectionnées de telle sorte que la teneur en eau ainsi que la teneur en oxygène soient toutes deux d'un maximum de 100 ppm.

8. Procédé de préparation de composés selon la revendication 5 ou 6, **caractérisé en ce qu'**un composé de formule (II)
(CₘF₂ₘ₊₁)Li (II),
où
m dans chaque cas, indépendamment les uns des autres, désigne 2, 3 ou 4, est, dans une première réaction, réagi avec du dichloroarylbismuthane ou du chloro(diaryl)bismuthane, où aryle dans le bismuthane correspondant désigne un groupement aryle ayant de 6 à 10 atomes de C, pouvant être substitué ou non substitué,
et les intermédiaires de formule (IIIa) ou (IIIb)
ArBi(CₘF₂ₘ₊₁)₂ (IIIa)
ou
Ar₂Bi(CₘF₂ₘ₊₁) (IIIb),
sont ensuite réagis avec du chlorure d'hydrogène, du bromure d'hydrogène, du HF anhydre ou du CF₃SO₃H, afin de donner les composés de formule (I), où Ar dans chaque cas, indépendamment les uns des autres, désigne un groupement aryle ayant de 6 à 10 atomes de C, pouvant être substitué ou non substitué, et où les conditions des réactions avec le composé de formule (II) sont sélectionnées de telle sorte que la teneur en eau ainsi que la teneur en oxygène soient toutes deux d'un maximum de 100 ppm.

9. Utilisation selon l'une ou plusieurs parmi les revendications 1 à 4 dans une réaction catalysée par un acide de Lewis choisie parmi une réaction de condensation, une alcoolyse, une réaction d'aldol, une réaction d'aldol de Mukaiyama, une réaction de Gattermann-Koch, un réarrangement de Beckmann et Fries, une acylation de Friedel-Crafts, une alkylation de Friedel-Crafts, une réaction de Mannich, une réaction de Diels-Alder, une réaction d'aza de Diels-Alder, une réaction de Baylis-Hillman, une réaction de Reformatsky, un réarrangement de Claisen, une réaction de cyclisation de Prins, une allylation de composés de carbonyle, une cyanation d'aldéhydes et de cétones, une cyanosilylation d'aldéhydes et de cétones, une cycloaddition 1,3-dipolaire ou une réaction de Michael.

10. Composés de formules (IIIa) et (IIIb)
ArBi(CₘF₂ₘ₊₁)₂ (IIIa),
Ar₂Bi(CₘF₂ₘ₊₁) (IIIb),
où
m dans chaque cas, indépendamment les uns des autres, désigne 2, 3 ou 4 et
Ar dans chaque cas, indépendamment les uns des autres, désigne un groupement aryle ayant de 6 à 10 atomes de C, pouvant être substitué.

11. Utilisation d'au moins un composé selon la revendication 10, comme catalyseur d'acide de Lewis.
